(19) Europäisches Patentamt
European Patent Office
Office européen des brevets

(11) Veröffentlichungsnummer: **0 111 189**
**B1**

(12) # EUROPÄISCHE PATENTSCHRIFT

(45) Veröffentlichungstag der Patentschrift: **26.02.86**

(51) Int. Cl.⁴: **C 07 D 251/34**, C 08 G 18/72, C 08 G 18/79, C 07 C 118/00

(21) Anmeldenummer: **83111544.9**

(22) Anmeldetag: **18.11.83**

(54) **Neue Polyisocyanatgemische, ein Verfahren zu ihrer Herstellung und ihre Verwendung als Aufbaukomponente bei der Herstellung von Polyurethankunststoffen.**

(30) Priorität: **01.12.82 DE 3244407**

(43) Veröffentlichungstag der Anmeldung: **20.06.84 Patentblatt 84/25**

(45) Bekanntmachung des Hinweises auf die Patenterteilung: **26.02.86 Patentblatt 86/9**

(84) Benannte Vertragsstaaten: **BE DE FR GB IT**

(56) Entgegenhaltungen:
**DE - A - 1 929 034**
**DE - A - 2 063 731**
**DE - B - 1 022 789**
**FR - A - 2 290 459**

(73) Patentinhaber: **BAYER AG, Konzernverwaltung RP Patentabteilung, D-5090 Leverkusen 1 Bayerwerk (DE)**

(72) Erfinder: **Richter, Roland, Dr., Roggendorfstrasse 53, D-5000 Köln 80 (DE)**
Erfinder: **Hettel, Hans, Dr., Hahnenweg 5, D-5000 Köln 80 (DE)**
Erfinder: **Müller, HannsPeter, Dr., Im Kerberich 6, D-5068 Odenthal (DE)**

EP 0 111 189 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue, bei Raumtemperatur flüssige und lagerfähige, Isocyanuratgruppen aufweisende Polyisocyanate auf Basis von 2,4-Diisocyanatotoluol enthaltende Polyisocyanatgemische, ein Verfahren zu ihrer Herstellung und ihre Verwendung als Aufbaukomponente bei der Herstellung von Polyurethankunststoffen nach dem Isocyanat-Polyadditionsverfahren.

Isocyanuratmodifizierte Diisocyanato-toluole und ihre Verwendung zur Herstellung von Polyurethankunststoffen sind im Prinzip bereits bekannt (vgl. z.B. DE-OS 22 21 81 1, DE-OS 24 52 532, DE-OS 24 03 858, DE-OS 20 63 731, DE-AS 10 22 789 oder US-PS 42 43 756 (Beispiel 5)).

Den in diesen Vorveröffentlichungen konkret beschriebenen Isocyanurat-modifizierten Polyisocyanatgemischen auf Basis von Diisocyanato-toluol, insbesondere auf Basis der technischen 80/20-Mischung aus 2,4- und 2,6-Diisocyanatotoluol haftet der Mangel an, daß sie entweder keine bei Raumtemperatur lagerfähigen, homogene Flüssigkeiten darstellen, oder daß sie zwecks Gewährleistung dieser Eigenschaft erhebliche Mengen an stabilisierend wirkenden Zusätzen enthalten. Derartige stabilisierend wirkende Zusatzmittel sind beispielsweise Urethan- und/ oder Allophanatmodifizierte Polyisocyanate wie sie in situ durch Modifizierung der Polyisocyanate mit Polyolen vor oder nach der Trimerisierungsreaktion erhalten werden; andere Stabilisierungsmittel sind beispielsweise die Amid- und/oder Acylharnstoffgruppierungen aufweisenden Verbindungen gemäß DE-OS 24 03 858. Diesen, die Lagerstabilität erhöhenden Zusatzmitteln gemeinsam ist das vorliegen von aktiven, an Stickstoff gebundenen Wasserstoffatomen, die eine mehr oder minder stark ausgeprägte Tendenz zur Reaktion mit Isocyanatgruppen aufweisen. Demzufolge weisen die, die genannten Zusatzmittel enthaltenden Gemische zwar oftmals eine ausreichende Lagerstabilität jedoch keine NCO-Wert-Stabilität auf, da unkontrollierbare Sekundärreaktionen der Isocyanatgruppen mit den aktiven Wasserstoffatomen der Zusatzmittel nicht ausgeschlossen werden können.

Reine Isocyanurat-modifizierte Polyisocyanatgemische auf Basis von Diisocyanatotoluol, die bei Raumtemperatur homogene Flüssigkeiten darstellen, und die eine ausreichende Lagerstabilität besitzen, sind bislang nicht bekannt geworden. Unter ausreichender Lagerstabilität wird verstanden, daß die vor Luftfeuchtigkeit geschützten Polyisocyanate im Temperaturbereich von 20 bis 40 °C flüssig bleiben und über einen Zeitraum von mindestens 6 Monaten keine Trübungen oder Ausfällungen auftreten. Desweiteren muß durch Abkühlung auf < 0 °C durchkristallisierte Ware, in dem angegebenen Temperaturbereich stets wieder völlig klar aufschmelzen.

Es war daher die der vorliegenden Erfindung zugrundeliegende Aufgabe, bei Raumtemperatur flüssige und lagerstabile Isocyanurat-modif izierte Polyisocyanate auf Basis von Diisocyanatotoluol zur Verfügung zu stellen, die keine nennenswerten Mengen an Verbindungen mit aktiven Wasserstoffatomen enthalten, so daß die NCO-Wert-Konstanz beeinträchtigende, unkontrollierbare Nebenreaktionen ausgeschlossen werden können.

Diese Aufgabe konnte durch die Bereitstellung der nachstehend näher beschriebenen erfindungsgemäßen Polyisocyanatgemische und das zu ihrer Herstellung geeignete erfindungsgemäße Verfahren gelöst werden.

Gegenstand der Erfindung sind bei Raumtemperatur flüssige, lagerstabile, einen Isocyanatgehalt von 36,5 bis 45 Gew.-% aufweisende, Polyisocyanatgemische, dadurch gekennzeichnet, daß sie im wesentlichen aus einer Abmischung aus.

a) 25-70 Gew.-Teilen eines Gemischs mit einem NCO-Gehalt von 22-36 Gew.-% aus (i) Isocyanuratgruppen aufweisenden Trimerisaten des 2,4-Diisocya-natotoluols mit (ii) 2,4-Diisocyanatotoluol

und

b) 30-75 Gew.-Teilen unmodifiziertem 2,4- und/oder 2,6-Diisocyanatotoluol

bestehen.

Gegenstand der Erfindung ist auch ein Verfahren zur Herstellung der neuen Polyisocyanatgemische, welches dadurch gekennzeichnet ist, daß man

a) 25-70 Gew.-Teile eines teiltrimerisierten 2,4-Diisocyanatotoluols mit einem NCO-Gehalt von 22-36 Gew.-%, welches durch Trimerisierung eines Teils der Isocyanatgruppen von 2,4-Diisocyanatotoluol in Gegenwart eines Trimerisierungskatalysators hergestellt worden ist, mit

b) 30-75 Gew.-Teilen 2,4- und/oder 2,6-Diisocyanatotoluol

so vermischt, daß ein Polyisocyanatgemisch mit einem NCO-Gehalt von 36,5 bis 45 Gew.-% resultiert.

Gegenstand der Erfindung ist auch die Verwendung der neuen Polyisocyanatgemische als Aufbaukomponente bei der Herstellung von Polyurethankunststoffen nach dem I socyanat-Polyadditionsver fahren.

Das erfindungsgemäße Verfahren gestattet die Herstellung von bei Raumtemperatur als homogene Flüssigkeit lagerstabilem, Isocyanurat-modif iziertem Diisocyanatotoluol, wobei der Gehalt der Gemische an Verbindungen mit, an Stickstoff gebundenem aktivem Wasserstoff, berechnet als -NH- (Molekulargewicht = 15), unter 0,5 Gew.-% gehalten werden kann. Unter Verbindungen mit, an Stickstoff gebundenem, aktivem Wasserstoff sind hierbei beliebige Verbindungen zu verstehen, die, mit Isocyanatgruppen reaktionsfähige, HN-Gruppen aufweisen, insbesondere beliebige, HN-Bindungen aufweisende Harnstoffe, Acylharnstof

fe, Urethane und/oder Allophanate, wie sie bei den Verfahren des oben zitierten Standes der Technik als Zusatzmittel verwendet werden, bzw. wie sie aufgrund der Mitverwendung von Alkoholen bei den Verfahren des genannten Standes der Technik als Sekundärprodukte entstehen.

Beim erfindungsgemäßen Verfahren wird äls Ausgangsmaterial für die Trimerisierungsreaktion ausschließ-lich das technisch reine, d.h. einen Reinheitsgrad von mindestens 95 Gew.-%, vorzugsweise mindestens 98 Gew.-% aufweisende, einheitliche Isomere, 2,4-Diisocyanatotoluol, eingesetzt, da nur unter dieser Voraussetzung bei Raumtemperatur lagerfähige Flüssigkeiten als erfindungsgemäße Verfahrensprodukte erhalten werden. Die Tatsache, daß bei Verwendung der technisch üblichen 80/20 oder 65/35-Mischungen aus 2,4- und 2,6-Diisocyanatotoluol keine lagerstabilen Verfahrensprodukte erhalten werden, ist insofern überraschend, als bei der Trimerisierung von 2,4-Diisocyanatotoluol theoretisch nur vier verschiedene isomere Monoisocyanurate auftreten können:

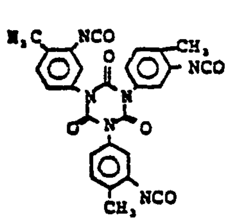

I

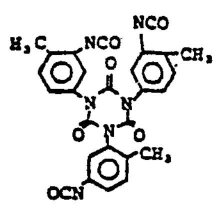

II

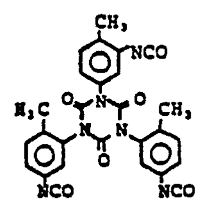

III

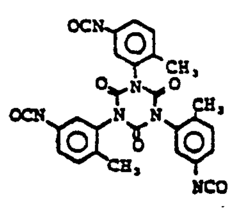

IV

Dagegen können bei Verwendung von Gemischen aus 2,4-und 2,6-Diisocyanatotoluol neben den bereits genannten Typen I bis IV zusätzlich noch die weiteren Typen V bis X auftreten, wodurch die Polymolekularität erhöht wird, so aß eine bessere Lagerstabilität erwartet werden sollte. Überraschenderweise bestätigten die erfindungsgemäß aufgefundenen Erkenntnisse jedoch in keiner Weise diese theoretischen Erwartungen.

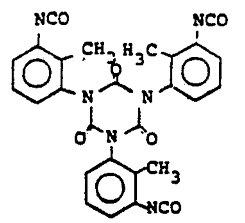

V

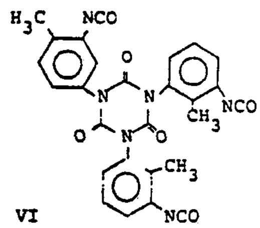

VI

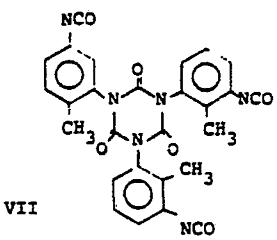

VII

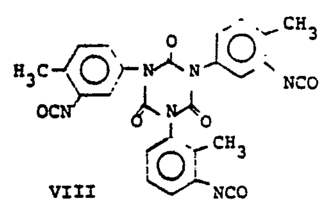

VIII

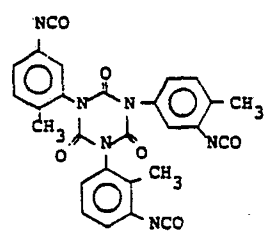

IX

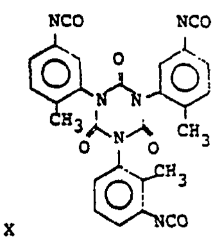

X

Das erfindungsgemäße Verfahren kann im Prinzip mit beliebigen Trimerisierungskatalysatoren des Standes der Technik durchgeführt werden, vorzugsweise werden jedoch die in den veröffentlichten europäischen Patentanmeldungen 0056158 und 0056159 beschriebenen Komplexe von Alkalimetallverbindungen als Trimerisierungskatalysatoren eingesetzt. Besonders bevorzugt ist das entsprechend den zuletzt genannten Veröffentlichungen mit offenkettigen oder cyclischen Polyethylenoxydpolyethern komplexierte Kaliumacetat.

Bei der Durchführung des erfindungsgemäßen

Verfahrens geht man im allgemeinen so vor, daß man 25 bis 70 Gew.-Teile technischen 2,4-Diisocyanatotoluols so lange einer Trimerisierungsreaktion unterzieht, bis der NCO-Gehalt von 48,25 Gew.-% auf 36-22 Gew.-% abgesunken ist. Dies entspricht einem Trimerisierungsgrad von ca. 25 bis 55% (Trimerisierungsgrad = Prozentsatz der im Ausgangsdiisocyanat vorliegenden Isocyanatgruppen, die unter Trimerisierung abreagieren). Nach Erreichen des gewünschten Trimerisierungsgrades wird der Katalysator vorzugsweise desaktiviert und das resultierende Gemisch mit 75-30 Gew.-Teilen 2,4-Diisocyanatotoluol, 2,6-Diisocyanatotoluol oder beliebigen Mischungen aus diesen Isomeren, vorzugsweise mit der technischen 80/20-Mischung der genannten Isomeren, vorzugsweise im Anschluß an die oder gleichzeitig mit der Zugabe des Katalysatorgifts so verdünnt, daß ein Gemisch mit einem NCO-Gehalt von 36,5-45 Gew.-% resultiert. Auf diese Weise sind klare, bei Raumtemperatur lagerstabile Lösungen von Isocyanurat-Gruppen aufweisendem Polyisocyanat auf Basis von 2,4-Diisocyanatotoluol in überschüssigen Diisocyanatotoluol zugänglich. Auch bei der Durchführung der Trimerisierungsreaktion bis zu einem Trimerisierungsgrad von 50-55% liegt als Produkt der Trimerisierungsreaktion noch ein Gemisch aus Trimerisaten des 2,4-Diisocyanatotoluols mit freiem 2,4-Diisocyanatotoluol vor, da bei der Trimerisierungsreaktion nicht nur Monoisocyanurate der oben angegebenen Formeln sondern auch 'Polyisocyanurate", d.h. Isocyanatgruppen aufweisende Verbindungen, die mehr als einen Isocyanuratring im Molekül enthalten, entstehen, so daß beispielsweise bei einem Trimerisierungsgrad von 55 % im Reaktionsgemisch noch ca. 15 Gew.-% an freiem 2,4-Diisocyanatotoluol vorliegen. Dies bedeutet, daß der oben genannte NCO-Gehalt der Komponente a) von 22-36% einen Gehalt an freiem 2,4-Diisocyanatotoluol von ca. 15 bis ca. 65 Gew.-% entspricht.

Zur Durchführung der erfindungsgemäßen Trimerisierungsreaktion wird das 2,4-Diisocyanatotoluol im allgemeinen bei 20 bis 120 °C, vorzugsweise 40 bis 80 °C vorgelegt und mit 5 bis 50 ppm, vorzugsweise 10 bis 30 ppm des Katalysators (beispielsweise mit einer äquimolaren Menge eines Polyethylenglykols des mittleren Molekulargewichts 370 komplexiertem Kaliumacetat) versetzt. Die Katalysatormenge wird im allgemeinen so bemessen, daß die Reaktionstemperatur langsam auf 85 bis 160 °C, vorzugsweise 100 bis 140 °C und besonders bevorzugt auf 110 bis 130 °C ansteigt. Bei Erreichung dieser optimalen Reaktionstemperatur hält man den Ansatz durch äußeres Heizen bzw. Kühlen temperaturstabil und läßt die Reaktion weiterlaufen, bis der gewünschte NCO-Gehalt erreicht ist.

Dann wird die Trimerisierungsreaktion vorzugsweise durch Zugabe einer dem Kaliumacetat mindestens äquimolaren Menge eines Katalysatorgifts abgestopt.

Geeignete Katalysatorengifte sind z.B. Säurechloride wie Acetylchlorid, Benzoylchlorid oder Carbaminsäurechloride oder Säuren wie Phosphorsäure, Chlorwasserstoff oder p-Toluolsulfonsäure.

Die Abmischung mit der Zweitkomponente der erfindungs-gemäßen Gemische, d.h. mit dem 2,4- und/oder 2,6-Di-isocyanatotoluol erfolgt vorzugsweise unmittelbar im Anschluß an die Beendigung der Trimerisierungsreaktion, d.h. innerhalb des Temperaturbereichs von 100 bis 140 °C, wobei sich das Gesamtgemisch im allgemeinen auf ca. 80 bis 1 00 °C abkühlt. Man rührt bei dieser Temperatur weiter, bis eine klare Lösung entstanden ist. Grundsätzlich ist es auch möglich, den Abbruch der Trimerisierungsreaktion und die Verdünnung mit unmodifiziertem Diisocyanatotoluol gleichzeitig dergestalt vorzunehmen, daß man das Reaktionsgemisch der Trimerisierungsreaktion mit einem Gemisch aus Diisocyanatotoluol und Katalysatorengift versetzt. Auch ein Abbruch der Trimerisierungsreaktion unmittelbar nach dem Verdünnen ist grundsätzlich möglich, jedoch weniger bevorzugt. Es ist auch denkbar, die Trimerisierungsreaktion ohne Verwendung eines Katalysatorgifts durchzuführen. Ein derartiger Verzicht auf die Mitverwendung eine s Katalysatorgifts ist dann möglich, wenn der Trimerisierungskatalysator in einer unteräquivalenten Menge, bezogen auf den Gehalt des eingesetzten 2,4-Diisocyanatotoluols an hydrolysierbaren Chlor, eingesetzt wird, so daß während der Trimerisierungsreaktion eine 'automatische" Desaktivierung des Katalysators erfolgt. Ein Verzicht auf die Mitverwendung von Katalysatorgiften ist selbstverständlich auch bei Verwendung der bekannten, sich unter dem Einfluß von Hitze zersetzenden Trimerisierungskatalysatoren möglich.

Die erfindungsgemäßen Polyisocyanatgemische eignen sich hervorragend zur Herstellung von Polyurethankunststoffen, insbesondere zur Herstellung von weichelastischen Polyurethanschaumstof fen eines (im Vergleich zur Verwendung von unmodif iziertem Diisocyanatotoluol) verbesserten Druckverformungsrestes und einer verbesserten Stauchhärte, wobei wegen der ausgezeichneten NCO-Wert-Stabilität der erfindungsgemäßen Gemische eine erhöhte Verschäumungssicherheit gewährleistet ist.

In den nachfolgenden Vergleichsbeispielen 1 bis 3 wird gezeigt, daß bei Verwendung einer technischen 80-20-Mischung aus 2,4- und 2,6-Diisocyanatotoluol bei der Trimerisierungsreaktion unabhängig vom Trimerisierungsgrad kein lagerstabiles Produkt erhalten wird:

**Beispiel 1 (Vergleich)**

In einem 2-ltr. Kolben mit Rührer, Stickstoffbegasung, Thermometer und Rückflußkühler legt man 400 g eines technischen 80/20-Gemischs aus 2,4- und 2,6-Diisocyanatotoluol bei 50 °C vor und versetzt mit 1,2 ml einer 0, 1 molaren Lösung von Kaliumacetat in Polyethylenglykol (mittl. MG: 370). Nach 12 Minuten ist die Innentemperatur auf 105°C angestiegen und der NCO-Gehalt auf 38,4 % gesunken. Durch Einrühren von 600 g einer 80/20-Mischung aus 2,4- und 2,6-Toluylendiisocyanat, versetzt mit 0,13 mmol Benzoylchlorid, wird die Trimerisierung abgebrochen. Man rührt bei 90 bis 100 °C weiter, bis alles Trimerisat klar gelöst ist. Das Endprodukt weist einen NCO-Gehalt von 44,3 % auf.

**Beispiel 2 (Vergleich)**

Analog Beispiel 1 erhält man durch Zugabe von 1,3 ml einer 0, 1 molaren Lösung von Kaliumacetat/18-Krone-6 (1:1) in Diethylenglykolmonomethylether ('18-Krone-6" = 1,4,7,10,13,16-Hexaoxacyclooctadecan,) zu 400 g einer 80/20-Mischung aus 2,4- und 2,6-Diisocyanatotoluol nach 15 Minuten eine Innentemperatur von 99 °C und einen NCO-Gehalt von 28,9 %, bei welchem die Reaktion durch vermischen mit 600 g einer 80/20-Mischung aus 2,4- und 2,6-Toluylendiisocyanat, versetzt mit 1,4 mmol Benzoylchlorid, abgebrochen wird. Das klar gelöste Endprodukt weist einen NCO-Gehalt von 40,5 % auf.

**Beispiel 3 (Vergleich)**

Analog Beispiel 1 erhält man durch Zugabe von 1,4 ml einer 0, 1 molaren Lösung von Kaliumacetat/18-Krone-6 (1:1) in Diethylenglykolmonomethylether zu 400 g einer 80/20-Mischung aus 2,4- und 2,6-Toluylendiisocyanat nach 9 Minuten eine Innentemperatur von 135°C und einen NCO-Gehalt von 22,4 %, bei welchem die Reaktion durch Vermischen mit 600 g einer 80/20-Mischung aus 2,4- und 2,6-Toluylendiisocyanat, versetzt mit 1,5 mmol Benzoylchlorid, abgebrochen wird. Das klar gelöste Endprodukt weist einen NCO-Gehalt von 37,9 % auf.

Das Produkt aus Vergleichsbeispiel 1 (Trimerisierungsgrad (TG) = 20,4 %) beginnt nach 11 Tagen, das Produkt aus Vergleichsbeispiel 2 (TG = 40 %) beginnt nach 14 Tagen und das Produkt aus Vergleichsbeispiel 2 (TG = 53,6 %) beginnt nach 16 Tagen einen kristallinen Niederschlag abzyscheiden. Läßt man alle drei Produkte bei T = -20°C völlig durchkristallisieren und schmilzt dann bei 30 °C wieder auf, so behalten alle drei eine Trübung und nach 2-3 Tagen fällt ein kristalliner Niederschlag aus.

**Allgemeine Herstellungsvorschrift für Patentbeispiele 4-10** (siehe Tabelle 1)

In einer Rührapparatur mit Thermometer, Rückflußkühler und Stickstoffbegasung legt man die angegebene Menge 2,4-Diisocyanatotoluol (2, 4-TDI) bei der angegebenen Starttemperatur ($T_s$) vor und versetzt mit der entsprechenden Katalysatorlösung

A = 0, 1 molare Lösung von Kaliumacetat/18-Krone-6 in Diethylenglykolmonomethylether

B = 0,025 molare Lösung von Kaliumacetat/Polyethylenglykol (mittl. MG = 370) (1:5) in Diethylenglykolmonomethylether

C = 0,025 molare Lösung von Kaliumacetat in Polyethylenglykol (mittl. MG = 370).

Danach rührt man bis der gewünschte Trimerisierungsgrad (TG) erreicht ist, wobei bei einem Temperaturanstieg über 130 °C die Trimerisierung sofort gestoppt wird, bzw. bei langsamen Temperaturanstieg auf 100 bis 130 °C der Ansatz durch zusätzliches Heizen auf der erreichten Maximaltemperatur ($T_h$) gehalten wird. Eine Abkühlung auf Temperaturen unter 100° C ist bei einem NCO-Gehalt < 32 % zu vermeiden, da bei einem solchen NCO-Gehalt ansonsten infolge starken Viskositätsanstiegs keine einwandfreie Durchmischung gewährleistet ist. Wenn der NCO-Gehalt auf den erforderlichen Wert abgesunken ist (Reaktionszeit $t_R$), wird die Trimerisierung durch Zugabe der angegebenen Menge einer 80/20-Mischung aus 2,4- und 2,6-Toluylendiiso-cyanat (80/20-2,4-, 2,6-TDI), versetzt mit einer dem verwendeten Kaliumacetat äquivalenten Menge Benzoylchlorid, abgestoppt und die Mischung bei 90-100°C weitergerührt bis alles Trimerisat klar gelöst ist. Die Daten der erhaltenen Produkte aus den Beispielen 4 bis 9 gibt die nachfolgende Tabelle wieder:

**Tabelle**

| | Menge (g) 2,4-TDI | $T_s$ (°C) | Katalysator Typ Lsg. (ml) | $T_h$ (°C) | $t_R$ (min.) | NCO (%) | Menge 80/20-2,4/2,6-TDI (g) | Endprodukt NCO (%) |
|---|---|---|---|---|---|---|---|---|
| Beispiel 4 | 800 | 50 | C; 3,4 | 130 | 2 | 33,1 | 1200 | 42,2 |
| Beispiel 5 | 800 | 80 | C; 3,7 | 121 | 115 | 29,1 | 1200 | 40,6 |
| Beispiel 6 | 800 | 70 | B; 5,8 | 105 | 70 | 30,4 | 1200 | 41,1 |
| Beispiel 7 | 100 | 50 | A; 0,3 | 96 | 10 | 35,2 | 150 | 43 |
| Beispiel 8 | 400 | 50 | A; 1,2 | 159 | 4 | 23,3 | 600 | 38,3 |
| Beispiel 9 | 2000 | 50 | C; 6,2 | 130 | 30 | 29,6 | 3000 | 40,8 |

Die Produkte der Patentbeispiele 4-9 zeigen eine Lagerstabilität, die größer als 6 Monate ist. Durch Abkühlung auf T = -20 °C völlig durchkristallisiertes Produkt schmilzt im Temperaturbereich 20-40° C wieder rückstandsfrei zu einer klaren Lösung.

**Beispiel 10 (Verwendungsbeispiel)**

Ein Gemisch bestehend aus eines Polyetherpolyols der OH-Zahl 28, hergestellt durch

100 Gew.-Teilen propoxylierung von Trimethylolpropan und anschließende Ethoxylierung des Propoxylierungsprodukt (PO:EO-Gewichtsverhältnis = 87:13)

3,2 Gew.-Teilen Wasser

0,2 Gew.-Teilen Triethylendiamin

0,1 Gew.-Teilen Bis- (dimethylaminoethyl) -ether

1,6 Gew.-Teilen Diisopropanolamin

1,0 Gew.-Teilen Diethanolamin

0,15 Gew.-Teilen eines handelsüblichen chlorhaltigen Polysiloxanstabilisators (Stabilisator K5 53, Bayer AG, Leverkusen)

3,9 Gew.-Teilen Glycerin

wird mit 65,5 Gew.-Teilen eines gemäß Beispiel 5 hergestellten Isocyanates intensiv vermischt und in einer offenen Form verschäumt. Es entsteht ein nicht schrumpfender, leicht aufdrückbarer hochelastischer Schaumstoff mit folgenden mechanischen Eigenschaften:

Rohdichte (kg/m³) 38
Zugfestigkeit (kPa) 95
Bruchdehnung (%) 120
Stauchhärte (kPa) 4,6
bei 40 % Verformung
Druckver formungsre st (%) 7, 5
bei 90 % Kompression

**Patentansprüche**

1. Bei Raumtemperatur flüssige, lagerstabile, einen Isocyanatgehalt von 36,5 - 45 Gew.-% aufweisende Polyisocyanatsgemische, dadurch gekennzeichnet, daß sie im wesentlichen aus einer Abmischung aus

a) 25 - 70 Gew.-Teilen eines Gemischs mit einem NCO-Gehalt von 22-36 Gew.-% aus (i) Isocyanuratgruppen aufweisenden Trimerisaten des 2,4-Diisocyanatotoluols mit (ii) 2,4-Diisocyanatotoluol

und

b) 30-75 Gew.-Teilen unmodifiziertem 2,4- und/oder 2,6-Diisocyanatotoluol

bestehen.

2. Polyisocyanatgemisch gemäß Anspruch 1, dadurch gekennzeichnet, daß es einen Gehalt an Verbindungen mit an Stickstoff gebundenen aktiven Wasserstoffatomen, berechnet als -NH-, von weniger als 0,5 Gew.-% aufweist.

3. Verfahren zur Herstellung von Polyisocyanatgemischen gemäß Ansprüchen 1 und 2, dadurch gekennzeichnet, daß man

a) 25-70 Gew.-Teile eines teiltrimerisierten 2,4-Diisocyanatotoluols mit einem NCO-Gehalt von 22-36 Gew.-%, welches durch Trimerisierung eines Teils der Isocyanatgruppen von 2,4-Di = isocyanatotoluol in Gegenwart eines Trimerisierungskatalysators hergestellt worden ist, mit

b) 30-75 Gew.-Teilen 2,4- und/oder 2,6-Diisocyanatotoluol

so vermischt, daß ein Polyisocyanatgemisch mit einem NCO-Gehalt von 36,5-45 Gew.-% resultiert.

4. Verfahren gemäß Anspruch 3, dadurch gekennzeichnet, daß man als Trimerisierungskatalysator Kaliumacetat in mit offenkettigen oder cyclischen Polyethylenoxidpolyethern komplexierter Form verwendet.

5. Verwendung der Polyisocyanatgemische gemäß Anspruch 1 und 2 als Aufbaukomponente bei der Herstellung von Polyurethankunststoffen nach dem Isocyanat-Polyadditionsverfahren.

## Claims

1. Polyisocyanate mixtures which are liquid and storage-stable at room temperature and which have an isocyanate content of 36.5 - 45% by weight, characterised in that they consist essentially of a mixture of

a) 25 - 70 parts by weight of a mixture having an NCO content of 22-36% by weight of (i) isocyanurate-group-containing trimers of 2,4-diisocyanato-toluene and (ii) 2,4-diisocyanato-toluene and

b) 30-75 parts by weight of unmodified 2,4- and/or 2,6-diisocyanato-toluene.

2. Polyisosyanate mixture according to Claim 1, characterised in that it contains less than 0.5% by weight of compounds containing active hydrogen atoms attached to nitrogen, expressed as -NH-.

3. Process for producing polyisocyanate mixtures according to Claims 1 and 2, characterised in that

a) 25-70 parts by weight of a partly trimerised 2,4-diisocyanato-toluene with an NCO content of 22-36% by weight which has been produced by trimerising some of the isocyanate groups of 2,4-diisocyanato-toluene in the presence of a trimerisation catalyst,

are mixed with

b) 30-75 parts by weight of 2,4- and/or 2,6-diisocyanato-toluene in such a way that a polyisocyanate mixture having an NCO content of 36.5-45% by weight is obtained.

4. Process according to Claim 3, characterised in that potassium acetate complexed with open-chain or cyclic polyethylene oxide polyethers is used as the trimerisation catatyst.

5. Use of the polyisocyanate mixtures according to Claim 1 and 2 as a synthesis component in the production of polyurethane plastics by the isocyanate polyaddition process.

## Revendications

1. Mélanges de polyisocyanates liquides à la température ambiante, stables à l'entreposage, présentant une teneur en isocyanate de 36,5 à 45 % en poids, caractérisés en ce qu'ils sont constitués essentiellement par un mélange de

a) 25 à 70 parties en poids d'un mélange à teneur en NCO de 22 à 36% en poids (i) de produits de trimérisation porteurs de groupes isocyanurate du 2,4-diisocyanato-toluène avec (ii) du 2,4-diisocyanatotoluène et

b) 30 à 75 parties en poids de 2,4- et/ou de 2,6-diisocyanatotoluènes non modifiés.

2. Mélange de polyisocyanates suivant la revendication 1, caractérisé en ce qu' il présente une teneur en composés à atomes d'hydrogène actif liés à l'azote, exprimés en -NH-, de moins de 0,5 % en poids.

3. Procédé de production de mélanges de polyisocyanates suivant les revendications 1 et 2, caractérisé en ce qu'on mélange

a) 25 à 70 parties en poids d'un 2,4-diisocyanatotoluène partiellement trimérisé à teneur en NCO de 22 à 36% en poids, qui a été préparé par trimérisation d'une partie des groupes isocyanato du 2,4-diisocyanatotoluène en présence d'un catalyseur de trimérisation, avec

b) 30 à 75 parties en poids de 2,4- et/ou de 2,6-diisocyanatotoluènes

de manière à former un mélange de polyisocyanates à teneur en NCO de 36,5 à 45% en poids.

4. Procédé suivant la revendication 3, caractérisé en ce qu' on utilise comme catalyseur de trimérisation l'acétate de potassium sous une forme complexée avec des polyéthylènoxyde-polyéthers à chaîne ouverte ou cycliques.

5. Utilisation des mélanges de polyisocyanates suivant les revendications 1 et 2 comme composant structural dans la production de matières plastiques du type polyuréthanne par le procédé de polyaddition d'isocyanates.